# EUROPEAN PATENT APPLICATION

(11) **EP 0 671 182 A1**
(43) Date of publication of application: **13.09.1995**
(21) Application number: 95103041.0
(22) Date of filing: 03.03.1995
(51) Int. Cl.: A61M 25/00

(54) **Catheter protector**

(30) Priority: 10.03.1994 SE 9400832
(71) Applicant: GAMBRO AB, S-220 10 Lund (SE)
(72) Inventor: Jerlöw, Agerbo, Grethe, DK-2000 Frederiksberg (DK)
(74) Representative: Asketorp, Göran

(57) **Abstract**

The catheter protector consists of a transparent plastic bag (7), with an upper hole (8) surrounded by an adhesive (9) and preferably one lower opening (11) which is closeable by means of a closure (12) of the zip type. The bag comprises a desiccant (14) for absorption of moisture within the bag. The bag is fastened to the skin surrounding a catheter entrance point (2) and protects the catheter connectors (3,4) between uses, whereby showering etc. is made possible.

## Description

### FIELD OF THE INVENTION

The present invention relates to a catheter protector for use in connection with permanent or semi-permanent catheters such as catheters for peritoneal dialysis or suclavia catheters or the like, for example epidural, pancreas or nephrostomy catheters. More particularly the invention concerns a bag intended to be applied around the catheter in order to protect it between uses and thus allow the patient to take a shower or to go swimming.

### PRIOR ART

The invention is primarily intended to be used in connection with catheters for hemodialysis or peritoneal-dialysis, for example catheters such as those disclosed in the patent documents EP-A1-0 504 934, US-A-5 057 073, US-A-5 053 003 and DE-A1-41 12 713.

Such a catheter passes through the skin and is often attached to the skin by means of several sutures. Additionally the catheter is provided with a coupling device for connection to tubes.

With hemodialysis the use of the catheter and the treatment occur relatively seldomly, for example every other day. Between these periods the patient normally wraps gauze bandage around the catheter and fastens it at a convenient place on the skin by means of plaster. This type of handling is naturally annoying for the patient and does not allow showering for example.

With peritoneal dialysis the catheter is used more often, for example four times a day or more. With this type of catheter the requirement for accessibility is greater and the packing-in of the catheter between uses is more awkward.

The disadvantages for these patient categories is that taking a bath is not possible, due to the danger of infection since the catheter protectors used today are not waterproof. Additionally, the protectors normally used are awkward and problematic for the patient and still result in a high risk of infection. The plasters used can bring about over-sensitivity reactions to the adhesive. The temporary bandages have to be removed each time the catheter is to be used, whereby the catheter entry point is easily irritated and these bandages are furthermore difficult and time-consuming to apply.

If a patient wants to take a shower, a plastic bag can be applied over the catheter in order to protect it against soap and water etc.

Plastic bags which are fastened to the skin are used in other areas, for example so-called colostomy bags. Such a bag comprises an upper opening intended to be attached to an attachment plate which is fixedly connected to the skin around the colostomy. As an alternative there are also colostomy bags which can be directly attached to the skin by means of adhesive.

Such a colostomy bag can be used directly without further modification in order to protect the catheter during showering for example.

If such a colostomy bag is used, certain disadvantages are immediately apparent. For example the bag is normally completely closed which means that the bag has to be removed every time the catheter is to be used. In this way the colostomy bag irritates the entry point of the catheter through the skin. The colostomy bag is additionally relatively bulky and heavy which unnecessarily loads the skin around the catheter entry point. The colostomy bag is normally opaque which is a disadvantage in the present case since the catheter cannot be inspected through the bag. Furthermore when using a colostomy bag it is soon noticed that the bag becomes coated with an inner layer of moisture due to the water vapour which is given off from the skin.

Examples of such a colostomy bag are disclosed in the patent documents EP-B1-0 078 930 and US-A-4 403 991.

### SUMMARY OF THE INVENTION

The object of the present invention is to give the aforementioned patient categories a higher quality of life by allowing the patient the possibility of taking a bath, a shower or to swim in a way that the patient no longer has to use the awkward temporary bandaging with gauze bandages and plasters which were used earlier with the risk of over-sensitivity reactions and so that the patient will experience a greater feeling of well-being by use of a catheter protector according to the present invention.

This is achieved in that the catheter protector is quick, easy and safe to position, in that the catheter bag is transparent so that the catheter entry point and the catheter can be inspected, in that the catheter protector allows access to the catheter during use, administration of medicine and blood testing, and in that the catheter bag minimizes the risk of infection which leads to a fewer number of catheter re-arrangements which have to be carried out in hospital. The risk of infection is a large problem especially with peritoneal catheters where infection of the peritonea means that the peritoneal dialysis can no longer be carried out or can only be carried out to a limited extent.

The object of the present invention is thus to provide a catheter protector which is specially designed in order to be suitable as a protector for catheters, particulary dialysis catheters.

A further object of the present invention is to achieve a catheter protector which is transparent so that the catheters can be observed through the protector and which is provided with means for removing moisture inside the bag.

A still further object of the present invention is to achieve a catheter protector which allows the catheter's connectors to be accessible without the bag having to be removed.

An additional object of the present invention is to achieve a catheter protector which is easily applied without irritating the catheter entry point through the skin.

According to the invention, a catheter protector is provided which meets the aforementioned objects and which has the features defined in the appended claims.

### DESCRIPTION OF THE DRAWINGS

The invention will now be described in more detail with respect to preferred embodiments of the invention and with reference to the accompanying drawings.

Fig. 1 is a perspective view of a first embodiment of the catheter protector according to the invention.

Fig. 2 is a frontal view of the catheter protector according to Fig. 1.

Fig. 3 is a rear view of a second embodiment of the catheter protector according to the invention.

Fig. 4 is a frontal view of a third embodiment of the catheter protector according to the invention in the form of a disposable bag.

Fig. 4a is a rear view of a catheter protector similar to the protector according to Fig. 4 but with a tear flap.

Fig. 4b is a rear view of the catheter protector according to Fig. 4 provided with protective paper for the adhesive.

Figs. 5-7 are frontal views of a fourth embodiment of the catheter protector according to the invention particularly adapted for peritoneal dialysis catheters.

Fig. 8 is a rear view of a fifth embodiment of the catheter protector according to the invention.

### DETAILED DESCRIPTION OF PREFERRED EMBODIMENTS

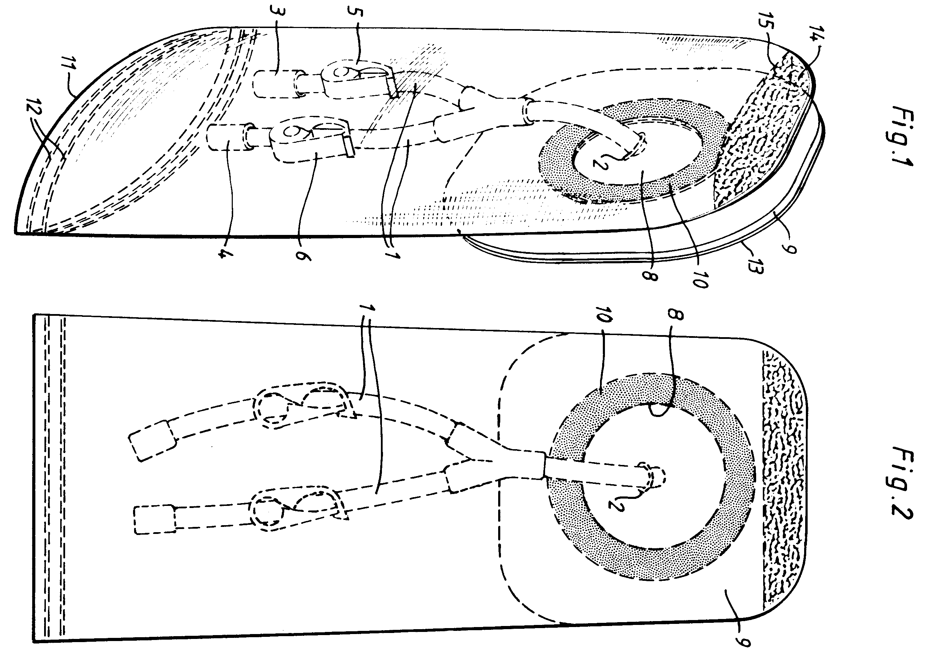

Fig. 1 shows a catheter protector according to the present invention in perspective.

A catheter 1 is shown in Fig. 1 with a suture 2 for attaching an entry device of the catheter through the skin. The catheter has an orifice for example in a blood vessel, or in the peritoneum or in another inner organ. The catheter 1 is provided at its other end with one or more connectors 3, 4 as well as clamps 5, 6 in a known manner. The entry device through the skin is naturally sensitive and has to be protected in the best manner against contamination or mechanical influences.

The catheter protector according to the invention consists of a substantially waterproof plastic bag 7 which, at its upper end, is provided with an opening 8. The opening 8 is surrounded by adhesive in the form of a disc 9 with a central opening corresponding to the opening 8. The plastic bag 7 is attached to the disc 9 with a circular ring-formed part 10. The disc 9 is intended to be connected to the skin with its side which is opposite to the bag 7.

The bag 7 according to Fig. 1 and Fig. 2 is further provided with an opening 11 in its lower side closed by means of zip-like closing means 12, for example in the form of a longitudinal bead running along one edge of the plastic bag and a corresponding recess on the other edge of the plastic bag. Such closing means are known.

The catheter protector according to the invention is used in the following way. The catheter bag is applied under the catheter and the outer end of the catheter with the connectors 3, 4 is fed through the hole 8, whereby the lower edge of the hole is suitably set tightly against the skin. The bag is threaded on the catheter until the opening 8 is positioned around the catheter entry point. After this, protective paper 13 on the outer side of the disc 9 is removed in order to expose the adhesive on the disc and the disc is pressed tightly against the skin around the catheter entrance point. The bag is thereby attached fixedly and in a waterproof manner around the catheter entrance point and effectively protects the catheter entrance point and the catheter.

When the catheter is to be used, the closure 12 at the lower side 11 of the bag 7 is opened. In this way the catheter connections 3, 4 are accessible through the opening 11 for connection to tubes of the medicinal apparatus which is to used, for example a dialysis machine. In order to ease the connection of the catheter with the tubes, the lower side 11 of the bag can be folded up so that the catheter connections 3, 4 are positioned under the lower end of the bag.

The hole 8 should be large enough to allow the catheter to be easily fed through the hole 8 without too much difficulty. The hole 8 can be circular as is shown in Fig. 1 and Fig. 2 or can have any other suitable shape such as oval or a rounded rectangular shape.

The catheter protector according to the invention is manufactured of plastic material, whereby at least the outer part of the bag is transparent so that the catheter and the catheter entrance point can be inspected through the bag. Suitable materials are polyvinyl chloride, PVC, or any polyolefine such as polyethylene or polypropylene. The bag should be soft and smooth and the choice of material is therefore made in accordance with this. In certain cases a plastic material which is opaque can be used or which is only partially transparent, such as a dull or frosted material.

The side of the bag directed towards the skin is suitably provided with a material which is repellant and soft against the skin so that the waterproof plastic bag itself will not contact the skin directly. One such suitable material would be a non-woven fibre plastic product, for example polypropylene or polyethylene.

The disc 9 is preferably manufactured of an adhesive with the property of being moisture-permeable so that the skin under the disc 9 can breathe. In this way it is avoided that the skin under the disc 9 becomes saturated and shrunken as well as irritated.

The part of the skin which surrounds the catheter entrance point and is positioned inside the hole 8 will give off moisture which results in mist on the inside of the bag. In order to take care of this, the bag is provided with a desiccant 14 arranged in a pocket 15, for example in the upper end of the bag. The desiccant is of a known type such as silicon gel and has the task of absorbing the moisture content in the bag. Other suitable materials are super absorbents. The pocket 15 is separated from the bag by means of a woven material which allows the passage of moisture.

The plastic bag 7 is attached to the disc 9 by means of a circular portion or a ring 10 with as small an area as possible. The reason for this is that the ring 10 will prevent moisture transport from the skin through the disc 9 to the atmosphere and produce a skin portion which is more easily irritated.

In order to prevent the area under the ring 10 from becoming irritated, the ring 10 can be provided with a plurality of holes so that the area of the ring 10 is perforated, whereby transport of moisture can occur through the disc 9 and the perforated ring 10 to the inside of the bag 7.

The size of the disc 9 is adapted so that the adhesive has sufficient grip to the skin and so that the forces are divided in a suitable manner. In Fig. 1 and Fig. 2 the disc 9 is relatively large and extends outside the ring 10.

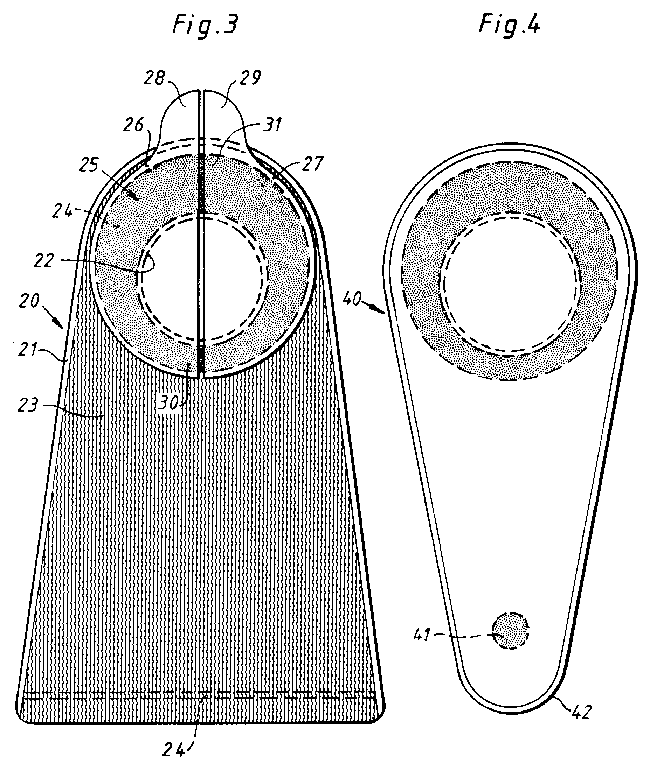

Fig. 3 shows a second embodiment of the catheter protector according to the invention. Fig. 3 thus shows a catheter bag 20 consisting of three welded-together plastic layers. The outer layer consists of a transparent tight plastic material, which does not let water through, such as PVC. The second layer should also be waterproof and can be of the same material as the first layer. The third layer which is directed towards the skin, consists of a non-woven fibre plastic product of polypropylene or polyethylene which is soft against the skin. The three plastic layers have the form shown in Fig. 3, i.e. two conical sides which are connected to each other at their upper ends along a radius and connected to each other at their lower end along a substantially straight line, which is however somewhat rounded. The plastic layers are connected to each other by means of a welded seam 21 so that a downwardly open bag is formed. The second and the third layer are provided with an upper circular opening 22 so that a connection with the inside of the bag is produced.

The material which is positioned against the skin is shown with crosshatching 23 in Fig. 3.

The lower end of the bag is closed with zip-like means 24. The lower end of the bag is slightly rounded so as to avoid sharp corners.

The opening 22 is surrounded by an adhesive mass 24 which surrounds the hole 22 and is attached to the intermediate layer. The layer 23 directed towards the skin only extends up to as far as the adhesive mass 24. The adhesive mass 24 is provided with protective paper 25 of known type on its surface which is directed towards the skin. The protective paper consists of two semicircles 26, 27, each of which is provided with a grip flap 28, 29. When the protective papers 26, 27 are to be removed, this occurs simply by gripping of the grip flaps 28, 29 and due to the fact that the protective paper is semicircularly formed.

Since the protective paper is semicircularly formed it allows the bag to be fed over the catheter and into position against the skin and held there, whereupon one half of the protective paper is removed and the adhesive allowed to fasten against the skin, whereupon the other half of the protective paper is removed and the bag is finally attached to the skin by pressing the adhesive against the skin. The adhesive can be adhesive which is activated by heat and/or pressure, or other suitable adhesive.

Other shapes of protective paper can of course be used, for example protective paper with two diagonal weakenings just opposite one another so that it can be torn into two halves during application. Another possibility is to use protective paper in the form of a ring with a tear indiction only at the upper end which ring is torn away during application and led around the contact surface of the bag against the skin.

As shown in Fig. 3 the adhesive mass 24 is formed so that the dimension of the lower part 30 is minimal whereas the dimension of the upper part 31 is significantly larger seen from the rear according to Fig. 3. The reason is that the minimal edge at the lower part 30 makes it easier to feed the catheter in through the hole 22 and to lead the bag over the catheter in place. The dimension of the upper end 31 is chosen to be sufficiently large in order to obtain the necessary adhesion to the skin. The size of the opening 22 is chosen such that the adhesive mass 24 is positioned well outside the area of the catheter entrance point which is easily irritated.

As is clear from Fig. 3 the bag is markedly conical which means that the lower end can be folded up like the rolling up of a shirt sleeve, so that the connections of the catheter are easily accessible. It is desirable to be able to use both hands during connection of the catheter to the tubes, which is simplified by the bag being able to be folded up in the manner described above.

The closure of the lower end of the bag 20 should preferably be waterproof in order to allow the patient to be able to shower as well as swim in a swimming pool. The closure can alternatively occur by folding the lower end of the bag and by the use of hook and loop tape in order to maintain the folding, or any other suitable connector such as press-studs, re-sealable tape etc. In certain cases re-sealable tape can completely replace the zip connector 24 according to Fig. 3.

As an alternative for the desiccant described in connection with Fig. 1 and Fig. 2, or together therewith, a moisture permeable membrane can be used, attached to the outside of the bag, for example right in front of the hole 22. Such a membrane is sold for example under the trademark GORE-TEX.

Such a membrane allows passage of moisture but is substantially waterproof. In this way the moisture inside the bag can be passed to the surroundings when the relative humidity of the surroundings is low (lower than 100%). With showering when the air humidity is high, a moisture layer can occur inside the bag but this disappears quickly when the air humidity of the surroundings falls. As an alternative, the second layer of the bag can consist of a membrane such that the moisture passes out via the second and the third layer. In this case it may be possible to leave out the third layer if the second layer is relatively skin-compatible. Additionally, an alternative is to apply non-woven material directly to the outer side of the second layer.

In certain cases it is possible to completely leave out the desiccant or the membrane, particularly if the bag is of the disposable type and is used only for a shorter time.

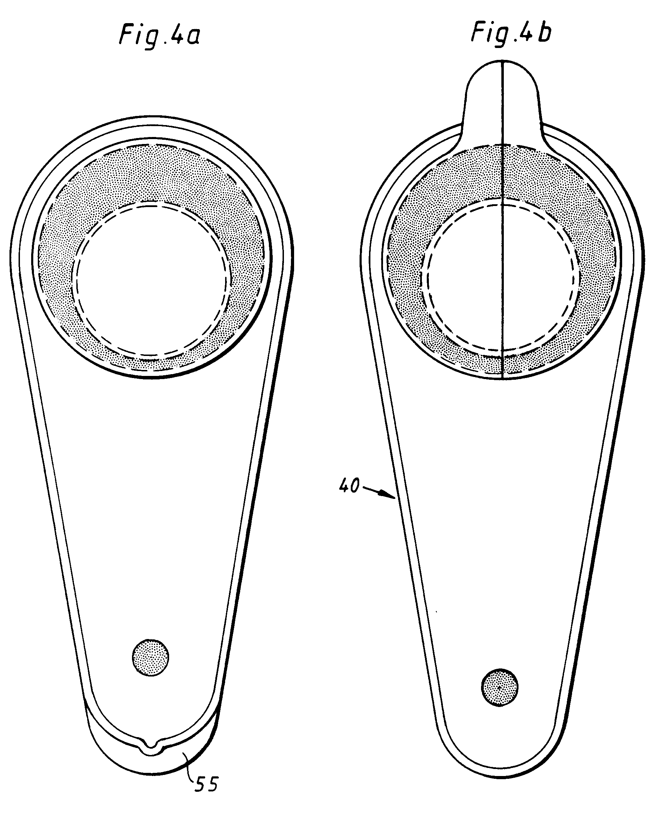

Fig. 4 shows a third embodiment of the catheter protector 40 according to the invention. This bag is of the disposable type and is used preferably with catheters for hemodialysis which are used relatively seldomly, for example about every other day. In use, the old catheter protector is removed and hemodialysis is carried out. After this the catheter is disinfected and a new catheter protector of the disposable type is applied. A catheter protector of the disposable type can be made lighter and more cheaply than the aforementioned recloseable catheter protector.

Fig. 4 shows that the catheter protector can be provided with a further attachment means 41 for fixing the lower part of the bag to the skin. In this way the catheter entrance point is protected additionally from mechanical influences from for example clothes, which tend to twist the catheter protector. The catheter protector 40 is relatively narrow at the lower end 42 in order to bunch together the catheter connectors. The disposable catheter protector according to Fig. 4 can be openable at the lower end 42 by tearing open a weakening if so desired, as is shown in Fig. 4a which depicts two flaps 55 for tearing open of the bag at the lower end.

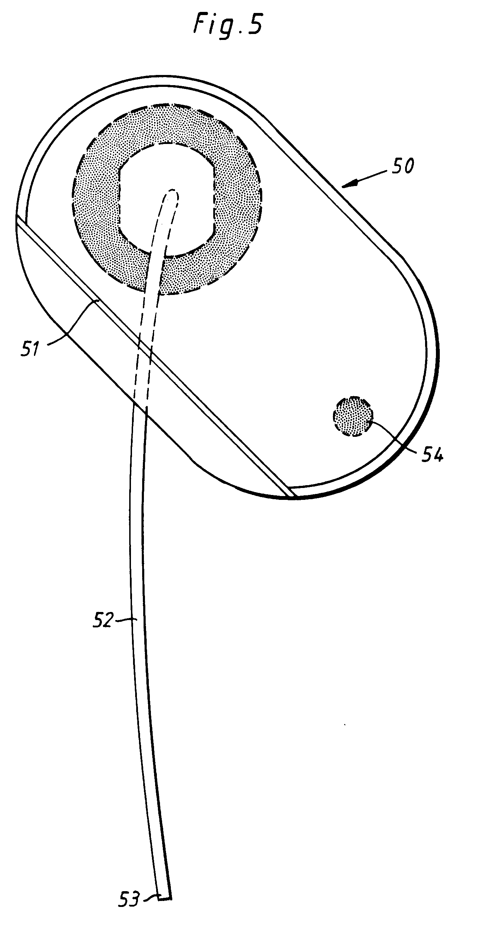

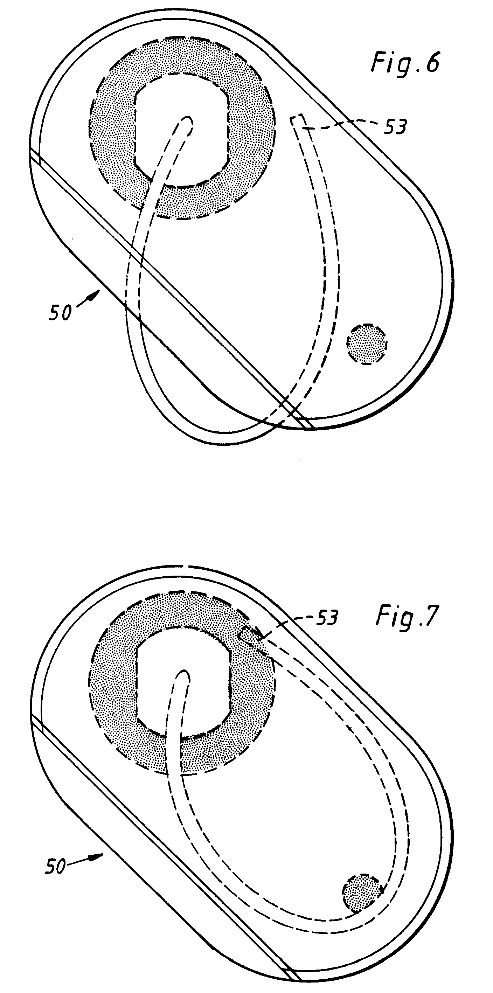

In Figs. 5-7 a fourth embodiment of the invention is shown, in which the catheter protector 50 is openable along one side. The bag can be closeable by means of a closure 51 of zip-type, such as has been described above, or by re-sealable tape, by folding or in any suitable manner. The reclosing should be completely waterproof in accordance with the invention.

As is disclosed in Fig. 5 the catheter bag 50 is applied so that it is inclined with respect to the catheter 52, which in this case is a peritoneal dialysis catheter. After use, the end 53 of the catheter is put back into the bag through the closure 51 so that the catheter 52 is nearly folded double. The orifice 53 is directed upwardly and positioned at a high level which is advantageous. The catheter bag for peritoneal dialysis is thereby small and convenient. The placement of the catheter bag 50 is further simplified if it is provided with an additional fastening means 54, as indicated in Fig. 5 with dashed lines.

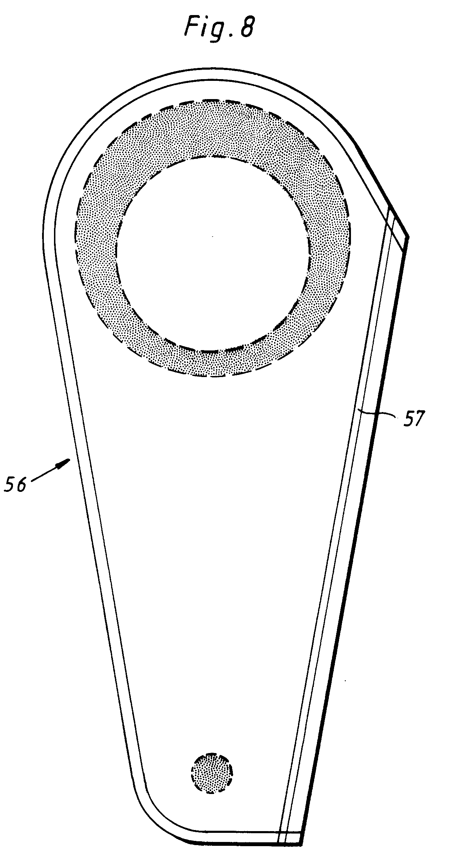

Finally Fig. 8 shows a catheter protector 56 provided with a closure 57 at the side. In other respects the catheter protector corresponds to the earlier described embodiments.

By applying the catheter in a closed plastic bag according to the invention, many advantages are obtained. The most direct advantage is that the patient is better able to take care of his hygiene since it is possible to shower. Additionally the handling of the catheter with dialysis is made easier since the gauze bandages used previously are no longer required.

An additional advantage is obtained in that the catheter can be cleaned and disinfected after use, after which a catheter bag according to the invention can be applied. In this way the catheter and the catheter entrance point are protected against attack by bacteria and the catheter can most likely be used for longer periods without being replaced which reduces the costs of hospital stay in connection with replacing a catheter. This effect is greater if the catheter bag remains dry internally since bacteria growth is appreciably lower in a dry environment, for which reason a desiccant or membrane are preferred. If the bag is only to be used for a short time the desiccant can however be left out.

The bag does not need to be sterilized during manufacture since the area which is to be protected is positioned on the outside of the skin. It is furthermore difficult to effectively sterilize an adhesive of the type which is used, at least in order to obtain any deep effect. The bag can however be provided with a sterilizing film on its inner surfaces, for example by spraying a sterilizing fluid onto the inside of the bag during or after manufacture. Additionally a sterilizing medium can be used during application of the bag around the catheter.

The catheter protector is intended to be used on catheters of permanent or semi-permanent type, which means times from a few days to several years. A peritoneal catheter can be used for several years while other types of catheters are replaced after a few weeks. A peritoneal catheter is typically accessed several times per day, while a catheter for ordinary hemodialysis is used and accessed each second day.

In this way a catheter protector has been achieved which meets the objects defined in the introduction. The catheter protector according to the invention can however be modified by a combination of the different characteristics which are disclosed in the different embodiments as well as in other ways which are obvious to the skilled man. Such modifications are of course included in the scope of the invention. The invention is limited only by the appended claims.

## Claims

1. Catheter protector for catheters of permanent or semi-permanent type arranged through the skin of a patient at an entry point, particularly a catheter (1) for dialysis, comprising a flexible bag (7) of substantially waterproof material; an aperture (8) in the bag surrounded by adhesive means (9) for attaching the bag to the skin of the patient around the catheter entry point thereby providing a substantially waterproof seal around the catheter, **characterized** in that the bag comprises reclosable closure means (12) for providing access to said catheter within the bag without removing the bag from the patient.

2. Catheter protector according to claim 1, **characterized** in that said closure means is positioned at the lower end of the bag and/or at the side edge of the bag.

3. Catheter protector according to claim 1 or 2, **characterized** in that the bag is provided with moisture removal means, such as a desiccant retained inside the bag and/or a membrane which is moisture permeable but substantially waterproof.

4. Catheter protector for catheters of permanent or semi-permanent type arranged through the skin of a patient at an entry point, particularly a catheter (1) for dialysis, comprising a flexible bag (7) of substantially waterproof material; an aperture (8) in the bag surrounded by adhesive means (9) for attaching the bag to the skin of the patient around the catheter entrance point thereby providing a substantially waterproof seal around the catheter, **characterized** in that the bag is provided with a moisture removal means (14) for controlling the moisture level within the bag.

5. Catheter protector according to claim 4, **characterized** in that the moisture removal means comprises a desiccant (14) retained inside the bag and/or a membrane which is moisture permeable but substantially waterproof.

6. Catheter protector according to any one of the preceding claims, **characterized** in that the adhesive means (9) is moisture-permeable.

7. Catheter protector according to any one of the preceding claims, **characterized** in that the bag is perforated at the contact surface (10) with the adhesive means.

8. Catheter protector according to any one of the preceding claims, **characterized** in that the adhesive means is narrower at the lower part (30) of the opening than at the upper part (31) of the opening.

9. Catheter protector according to any one of the preceding claims, **characterized** in that the bag is provided with a fastening means including adhesive means (41) for additional fixation of the bag.

10. Catheter protector according to any one of the preceding claims, **characterized** in that the adhesive means is provided with a protective paper being essentially ring shaped and divided or dividable into two half rings.
